# EUROPÄISCHE PATENTSCHRIFT

(11) **EP 3 684 515 B1**
(45) Veröffentlichungstag und Bekanntmachung des Hinweises auf die Patenterteilung: **21.07.2021**
(21) Anmeldenummer: 18769395.7
(22) Anmeldetag: 21.09.2018
(51) Int. Cl.: B01L 9/00, B01F 11/00, G01N 1/31, B01L 3/00

(54) **INKUBATIONSVORRICHTUNG SOWIE SYSTEM MIT INKUBATIONSVORRICHTUNG UND WIPPTISCH**
INCUBATION DEVICE AND SYSTEM COMPRISING INCUBATION DEVICE AND ROCKING PLATFORM
DISPOSITIF D'INCUBATION ET SYSTÈME ÉQUIPÉ D'UN DISPOSITIF D'INCUBATION ET TABLE BASCULANTE

(30) Priorität: 22.09.2017 EP 17192547
(43) Veröffentlichungstag der Anmeldung: 29.07.2020
(73) Patentinhaber: Euroimmun Medizinische Labordiagnostika AG, 23560 Lübeck (DE)
(72) Erfinder: KAFFKA, Christian, 23942 Dassow (DE); RATEIKE, Martin, 23689 Pansdorf Schleswig-Holstein (DE); STÖCKER, Winfried, 23627 Groß Grönau (DE)
(86) Internationale Anmeldenummer: PCT/EP2018/075614
(87) Internationale Veröffentlichungsnummer: WO 2019/057900

(56) Entgegenhaltungen:
- EP-A1- 2 191 893
- EP-A1- 3 085 446
- WO-A1-2014/009067
- SUSANNE LEMCKE ET AL: "Automated direct immunofluorescence analyses of skin biopsies : Automated DIF microscopy", JOURNAL OF CUTANEOUS PATHOLOGY., Bd. 43, Nr. 3, 1. März 2016 (2016-03-01), Seiten 227-235, XP055522745, DK ISSN: 0303-6987, DOI: 10.1111/cup.12637

## Beschreibung

Die vorliegende Erfindung betrifft eine Inkubationsvorrichtung zur pathohistologischen Untersuchung von biologischen Proben sowie ein System mit der Inkubationsvorrichtung und einem Wipptisch.

Eine biologische Probe ist vorzugsweise ein Gewebeschnitt, besonders vorzugsweise ein Gewebeschnitt von Humangewebe, ein Zellschnitt oder ein Protein.

Pathologische und labordiagnostische Untersuchungen stellen eine unverzichtbare Grundlage für die moderne Medizin dar. Mittlerweile steht eine Vielzahl von routinemäßig durchführbaren Tests zur Verfügung, mit Hilfe derer in Abwesenheit des Patienten aus Probenmaterial entscheidende Informationen zum vorliegenden Krankheitsbild, zur Prognose oder zum Erfolg einer Behandlung erhalten werden können.

Dabei können Patientenproben in Form von Gewebeschnitten oder Zellen zunächst angefärbt und die angefärbten Strukturen im Rahmen einer Befunderstellung dann untersucht werden. Insbesondere im Bereich der Histologie bzw. Histopathologie werden mikrometerdünne, gefärbte Gewebsschnitte hergestellt und am Mikroskop beurteilt.

Zum Probengut beim histologischen Arbeiten gehören vor allem Operationspräparate, Probeexzisionen sowie mittels Biopsien entnommenes Gewebe, wobei das vorrangige Ziel bei der Untersuchung derart angefärbter Gewebeschnitte in der sicheren Erkennung und Typisierung von Tumoren liegt. Mit Hilfe derartiger Verfahren können Gewebe histologisch charakterisiert und über die Untersuchungen von Wucherungen und Tumoren Krebs diagnostiziert werden.

Alternativ können Patientenproben auf das Vorhandensein oder die Konzentration bestimmter Moleküle untersucht werden, wenn die erhaltenen Werte anhand von Referenzdaten für die Diagnose nützliche Information darstellen. So kann der Nachweis von spezifischen Autoantikörpern anzeigen, dass der Patient unter einer Autoimmunkrankheit leidet. Beispielhafte Autoimmunkrankheiten umfassen entzündliche Krankheiten wie rheumatoide Krankheiten, Stoffwechselkrankheiten wie Diabetes und neurologische Erkrankungen.

Ein ungelöstes Problem ist die Knappheit und Hochpreisigkeit von Reagenzien und Probenmaterial. Wirtschaftlichkeitserwägungen führen dazu, dass Arbeitsabläufe und Materialverbrauch optimiert werden. Insbesondere geht der Trend in Richtung Miniaturisierung: diagnostische, pathohistologische und analytische Reaktionen werden nicht mehr im Maßstab von Millilitern, sondern von Mikro- oder gar Nanolitern ausgeführt. Das spart Reagenzien, Platz und gestattet, dass eine einmal gewonnene Probe genug Ausgangsmaterial für eine große Anzahl von diagnostischen Untersuchungen liefert. Nicht zuletzt bleibt dem Patienten auch für den Fall, dass ein einzelner diagnostischer Test misslingt, die erneute Entnahme einer Probe erspart.

Ein besonderes Problem bei der Miniaturisierung besteht darin, dass das Verhältnis des durch Kapillarkräfte und Adhäsion an Oberflächen zurückgehaltenen Flüssigkeitsvolumens einerseits und des Gesamtvolumens der für einen Verfahrensschritt eingesetzten Flüssigkeit andererseits besonders hoch ist. Mit anderen Worten haftet dermaßen viel Flüssigkeit an den Oberflächen, dass diese nach einem Verfahrensschritt durch Abgießen oder -pipettieren nicht effizient entfernt werden kann.

Es verbleibt ein relativ großes Volumen der Flüssigkeit, das nachfolgende Verfahrensschritte stört. Beispielsweise verdünnt eine verbliebene Waschlösung ein für eine nachfolgende Reaktion eingebrachtes Reagenz und verringert dadurch die Ausbeute bzw. Empfindlichkeit der nachfolgenden Reaktion.

Hierfür wurde in der europäischen Patentanmeldung EP 3085446 A1 eine Inkubationsrinne zum Inkubieren von einer biologischen Probe vorgeschlagen, in welche ein Träger mit der biologischen Probe eingelegt werden kann, um die biologische Probe in der Inkubationsrinne einer Inkubation mit einer oder mehreren Flüssigkeiten aussetzen zu können.

Des Weiteren ist es problematisch, wenn einzelne Proben individuell behandelt werden sollen oder müssen, insbesondere während andere Proben im selben Prozess geschwenkt bzw. gewippt werden. Eine individuelle Ansteuerung und Prozessierung von Proben ist bei pathohistologischen und labordiagnostischen Untersuchungen nur sehr umständlich oder gar nicht möglich.

US 2010/0124750 A1 beschreibt eine Vorrichtung zur Durchführung von immunologischen, histochemischen und zytochemischen, molekularbiologischen, enzymologischen, klinischchemischen und anderen Analysen, wobei die Vorrichtung einen Objektträger mit einem oder mehreren länglichen Klebeflächen und einen Reagenzhalter mit einem oder mehreren Kanälen aufweist.

WO 2016/169576 A1 beschreibt eine Inkubationsrinne mit einer von den Wänden der Inkubationsrinne gebildeten Vertiefung und einem Boden, wobei die Inkubationsrinne ein Mittel zum Absaugen von Flüssigkeit an wenigstens einem Längsende der Vertiefung aufweist, bevorzugt eine in einen Auslasskanal mündende Öffnung an dem Längsende der Vertiefung, besonders bevorzugt in einer Wand der Inkubationsrinne, wobei Öffnung und Auslasskanal so ausgeführt sind, dass Unterdruck angelegt werden kann, wobei die Inkubationsrinne über ihre Querachse schwenkbar ist, und wobei die Inkubationsrinne mit einem Träger bestückt werden kann.

WO2014/009067 offenbart eine Inkubationsvorrichtung mit mehreren Wipptischen, welche jeweils um eine jeweilige Achsen schwenkbar gelagert sind, wobei ein jeweiliger Wipptisch eine jeweilige Aufnahmevorrichtung für eine mechanisch reversible Aufnahme einer jeweiligen Inkubationsrinne aufweist. Hierbei wird ferner eine Antriebseinheit zum Bewirken einer Wippbewegung der Wipptische, insbesondere aus einer Nulllage heraus, offenbart, wobei jeder der jeweiligen Wipptische mit der Antriebseinheit über eine Mechanik gekoppelt ist.

Auch Lemke et al., J. Cutan. Pathol. 2016: 43: 227-235 (XP055522745) beschreibt eine Vorrichtung mit mehrere Wipptischen.

Der Erfindung liegt die Aufgabe zugrunde, eine Möglichkeit zur pathologischen oder labordiagnostischen Untersuchung von biologischen Proben bereitzustellen, wodurch eine individuelle Behandlung einzelner Proben ermöglicht wird und wobei eine Zeitersparnis und eine Erhöhung der Flexibilität erzielt werden können.

Diese Aufgabe wird durch die erfindungsgemäße Inkubationsvorrichtung gelöst.

Vorgeschlagen wird eine Inkubationsvorrichtung, welche eine Mehrzahl von Wipptischen aufweist, welche jeweils um eine jeweilige Achse, insbesondere eine Längsachse des Wipptisches, schwenkbar gelagert sind. Ein jeweiliger Wipptisch weist eine jeweilige Aufnahmevorrichtung für eine mechanisch reversible Aufnahme einer jeweiligen Inkubationsrinne auf. Ferner weist die Inkubationsvorrichtung eine gemeinsame Antriebseinheit zum gemeinsamen Bewirken jeweiliger gemeinsamer Wippbewegungen der jeweiligen Wipptische aus einer Nulllage heraus um einen bestimmten Winkelbereich aufgrund einer Antriebsbewegung der Antriebseinheit auf. Jeder der jeweiligen Wipptische ist mit der Antriebseinheit über eine jeweilige Koppelmechanik gekoppelt. Jede der jeweiligen Koppelmechaniken koppelt den jeweiligen Wipptisch mit der Antriebseinheit der Art elastisch federnd, dass bei Festhalten des Wipptisches der Wipptisch trotz Fortführung der Antriebsbewegnug in einer festen Position stehen bleibt, ferner jedoch nach Unterlassen des Festhaltens des Wipptisches der Wipptisch wieder aufgrund der Antriebsbewegung die Wippbewegung um den vorbestimmten Winkelbereich durchführt.

Insbesondere ist eine mechanische Kopplung eines bestimmten Wipptisches mit der Antriebseinheit mittels einer bestimmten Koppelmechanik unabhängig von einer anderen mechanischen Kopplung eines anderen Wipptisches mittels einer anderen Koppelmechanik an die Antriebseinheit.

Vorgeschlagen wird ferner ein mit einer erfindungsgemäßen Inkubationsvorrichtung sowie wenigstens eine in einen Wipptisch einlegbare Inkubationsrinne.

Die die Inkubationsrinne weist insbesondere eine von den Wänden der Inkubationsrinne gebildete Vertiefung mit einem Vertiefungsboden auf, wobei die Inkubationsrinne ferner insbesondere wenigstens eine in einen Kanal mündende Öffnung an einem Längsende der Vertiefung aufweist, bevorzugt in einer Wand der Inkubationsrinne, so dass über den Kanal Flüssigkeit in die Vertiefung eingebracht oder abgesaugt werden kann. Insbesondere kann in die Inkubationsrinne ein Träger eingelegt werden, welcher bei Einlegen in die Inkubationsrinne die Probe auf seiner Unterseite, welche dem Vertiefungsboden zugewandt ist, aufweist, so dass die Unterseite des Trägers, der Vertiefungsboden und die Wände ein Kompartiment für die Flüssigkeit bilden. Vorzugsweise weist die Inkubationsrinne Mittel zum Fixieren eines Abstandes zwischen der Unterseite des Trägers und dem Vertiefungsboden aufweist, so dass die Unterseite des Trägers, der Vertiefungsboden und die Wände ein Kompartiment für die Flüssigkeit bilden.

Um einen oder mehrere Vorteile, welche möglicherweise durch die Erfindung erreicht werden können, zu erläutern, folgen nun weitere Ausführungen.

Im Zuge von pathohistologischen Untersuchungen ist es im Laborbetrieb häufig der Fall, dass ein Träger mit einer biologischen Probe bzw. einem biologischen Gewebe zu einem ersten Zeitpunkt in einer ersten Inkubationsrinne eingelegt werden muss, um dann im Zuge eines sogenannten Waschprotokolls das Gewebe abfolgend in mehreren Waschschritten mit unterschiedlichen Waschflüssigkeiten in Kontakt zu bringen. Mitunter werden Inkubationsrinnen verwendet, in welche von oben her ein Objektträger mit der Probe auf der Unterseite des Objektträgers so eingelegt wird, dass die Probe sich gegenüber einem Boden der Inkubationsrinne befindet. Vorzugsweise wird hierbei ein Abstand zwischen der Probe und dem Boden durch Beabstandungselemente gewahrt, so dass durch diesen Abstand eine Kavität zwischen der Probe und dem Boden gebildet wird, innerhalb welcher sich eine Flüssigkeit befinden soll. Hierbei kann es vorkommen, dass sich unterhalb der Probe, also zwischen der Probe und dem Boden der Inkubationsrinne, Lufteinschlüsse befinden. Durch das Schwenken des Wipptisches wird dann ein Bewegen der Flüssigkeit herbeigeführt, so dass sich solche Lufteinschlüsse zu den Seiten der Inkubationsrinne hin verlagern und dort an entsprechenden Öffnungen der Inkubationsrinne austreten können. Auch hierdurch kann eine gleichmäßige Verteilung von Flüssigkeit auf der Probe gewährleistet werden. Um eine gleichmäßige Verteilung einer Waschflüssigkeit auf dem Gewebe gewährleisten zu können, ist ein Wipptisch schwenkbar gelagert, sodass die Flüssigkeit sich in der Inkubationsrinne während der Wippbewegung hin und her bewegen kann. Da eine Durchführung eines gesamten Waschprotokolls mit mehreren Waschschritten eine gewisse Zeitspanne einnimmt, kann es vorkommen, dass noch während einer Durchführung eines ersten Waschprotokolls für eine erste biologische Probe der Bedarf entsteht, eine andere biologische Probe mit einem anderen Waschprotokoll und dazugehörigen anderen Waschschritten zu prozessieren. Hierzu muss dann also zu einem späteren Zeitpunkt diese weitere biologische Probe in eine weitere Inkubationsrinne eingelegt werden, ggf. noch während der Durchführung des ersten Waschprotokolls für die erste Probe.

Eine Inkubationsrinne weist hierbei vorzugsweise eine Öffnung zum Einbringen von Waschflüssigkeiten als auch ein Öffnung zum Absaugen von Waschflüssigkeiten der einzelnen Waschschritte auf. Eine Inkubationsrinne, welche einmal für ein bestimmtes Waschprotokoll bzw. eine Abfolge von Waschschritten verwendet wurde, sollte aus Gründen von Kreuzeffekten bzw. Kontaminationen nicht sofort für eine andere biologische Probe verwendet werden. Daher ist es notwendig, dass eine Inkubationsrinne in einen Wipptisch eingelegt werden kann und auch wieder herausgenommen werden kann. Dieses wird durch die mechanisch reversible Aufnahme in die Aufnahmevorrichtung der erfindungsgemäßen Inkubationsvorrichtung ermöglicht. Sollen dann gegebenenfalls zu unterschiedlichen Zeitpunkten unterschiedliche Waschprotokolle für unterschiedliche biologische Proben durchgeführt werden, so kann im Laborbetrieb der Bedarf entstehen, zu unterschiedlichen Zeiten unterschiedliche Inkubationsrinnen in unterschiedliche Wipptische einzusetzen und gegebenenfalls auch wieder herauszunehmen.

Würde für eine jede einzelne Inkubationsrinne jeweils ein einzelner Wipptisch mit einer jeweils eigenen Antriebseinheit vorgesehen sein, so müsste für jede biologische Probe eine eigene Inkubationsvorrichtung mit einer eigenen Antriebseinheit und einem eigenen Wipptisch vorgesehen sein. Dadurch jedoch, dass die erfindungsgemäße Inkubationsvorrichtung eine Mehrzahl an Wipptischen aufweist, welche durch eine gemeinsame Antriebseinheit einer gemeinsamen, insbesondere gemeinsamen synchronen, Wippbewegung unterzogen werden, kann die erfindungsgemäße Inkubationsvorrichtung für die Prozessierung bzw. das Abarbeiten von Waschschritten jeweiliger Waschprotokolle für jeweilige biologische Proben verwendet werden. Hierzu muss dann lediglich jeweils für eine bestimmte biologische Probe eine entsprechende Inkubationsrinne in eine entsprechende Aufnahmevorrichtung eines entsprechenden Wipptisches eingelegt werden.

Es kann aus Kostengründen vorgesehen sein, dass eine gemeinsame Antriebseinheit die Wippbewegungen der jeweiligen Wipptische bewirkt, wobei jedoch für die Schritte des Einsetzens einer Inkubationsrinne in eine Aufnahmevorrichtung des Wipptisches oder auch das Herausnehmen einer Inkubationsrinne aus einer Aufnahmevorrichtung eines Wipptisches durch eine Laborkraft sehr viel Fingerspitzengefühl aufgebracht werden muss, um bei fortgeführter Wippbewegung eines Wipptisches die Inkubationsrinne einlegen bzw. herausnehmen zu können. Alternativ kann die Antriebseinheit für alle Wipptische zum Stillstand gebracht werden, um ungestört eine einzelne Inkubationsrinne einzulegen oder herauszunehmen. Hierdurch würden bestimmte Waschschritte für bestimmte Proben bestimmter Inkubationsrinnen bzw. Wipptische nicht mehr kontinuierlich durchgeführt werden und somit ggf. ein Prozessierungsergebnis verfälscht werden. Ferner ist es für eine ordnungsgemäße Abarbeitung von Waschprotokollen und der entsprechenden Waschschritte notwendig, zu unterschiedlichen, aufeinanderfolgenden Zeitpunkten aus einer Inkubationsrinne die dort vorhandene Waschflüssigkeit zu entfernen bzw. heraus zu holen, beispielsweise durch Absaugen, und dann für einen nächsten Waschschritt eine nächste, andere Waschflüssigkeit in die Inkubationsrinne einzubringen bzw. hinein zu pipettieren. Auch hierfür müsste eine Laborkraft sehr viel Fingerspitzengefühl aufbringen, wenn sie händisch bediente Instrumente für diese Tätigkeiten während einer fortgeführten Wippbewegung der Wipptische verwenden wollen würde. Dadurch jedoch, dass erfindungsgemäß für jeden Wipptisch eine Koppelmechanik vorgesehen ist, welche den jeweiligen Wipptisch mit der Antriebseinheit elastisch federnd koppelt, so dass bei Festhalten des Wipptisches der Wipptisch trotz Fortführung der Antriebsbewegung in einer festen Position stehen bleibt, ferner jedoch nach Unterlassen des Festhaltens des Wipptisches der Wipptisch wieder aufgrund der Antriebsbewegung die Wippbewegung um den bestimmten Winkelbereich durchführt, kann in vorteilhafter Weise auf einen Stillstand der Antriebseinheit und somit auf einen Stillstand der Gesamtheit der Wipptische verzichtet werden. Es kann entweder durch eine Laborkraft oder aber durch eine Haltevorrichtung eines Automaten ein bestimmter Wipptisch einfach festgehalten werden, ohne die Antriebsbewegung zum Herbeiführen der Bewegung der anderen Wipptische unterbrechen zu müssen. Es kann dann während des Festhaltens des Wipptisches beispielsweise eine Inkubationsrinne eingelegt oder herausgenommen werden. Es können dann aber auch während des Festhaltens Flüssigkeiten aus einer Inkubationsrinne rausgesaugt werden oder aber dort hinein pipettiert werden. Daher ist die erfindungsgemäße Inkubationsvorrichtung besonders vorteilhaft, denn sie erlaubt eine Prozessierung einer Mehrzahl von biologischen Proben auf einer Mehrzahl von Wipptischen bzw. entsprechenden Inkubationsrinnen, wobei die jeweiligen Waschprozeduren der jeweiligen biologischen Proben auf den jeweiligen Wipptisch nicht zu gleichen Zeitpunkten gestartet werden müssen, sondern zu unterschiedlichen Zeitpunkten beginnen als auch enden können. Es muss also nicht ein Waschprozess eines anderen Wipptisches bzw. einer anderen biologischen Probe in einer anderen Inkubationsrinne unterbrochen werden, wenn nur ein bestimmter Wipptisch bzw. eine bestimmte biologische Probe betroffen ist. Dies führt zu einer Erhöhung der Flexibilität als auch zu einer Zeitersparnis im Zuge einer Prozessierung mehrerer biologischer Proben durch jeweilige Waschschritte. Insbesondere können Proben zu unterschiedlichen Zeiten in den Gesamtprozess eingebunden bzw. aus dem Gesamtprozess ausgekoppelt werden.

Durch die elastisch federnde Kopplung zwischen der Antriebseinheit und dem Wipptisch kann ferner gewährleistet werden, dass für eine Laborkraft bei manuellem Eingreifen an einem bzw. in einen Wipptisch eine Gefahr des Einklemmens ihrer Finger zwischen dem Wipptisch und z.B. einem weiteren Wipptisch oder anderen Bauteilen vermieden wird, da der Wipptisch nicht starr an die Antriebseinheit gekoppelt ist. Hierdurch kann eine höhere Arbeitssicherheit für Laborkräfte gewährleistet sein.

Es wird also eine Möglichkeit bereitgestellt, eine individuelle Behandlung einzelner biologischer Proben zu ermöglichen. Vorteilhafterweise ist eine individuelle Behandlung einzelner biologischer Proben, insbesondere während andere biologische Proben auf anderen Wipptischen in entsprechenden Inkubationsrinnen geschwenkt werden, möglich. Es ist also vorteilhaft, dass eine Vielzahl solcher Inkubationsrinnen bzw. Wipptische gleichzeitig benutzt werden kann und dass diese individuell angesteuert werden können.

Vorteilhafte Ausführungsformen der Erfindung sind Gegenstand der abhängigen Ansprüche und werden in der folgenden Beschreibung unter teilweise Bezugnahme auf die Figuren näher erläutert.

Vorzugsweise weist die Koppelmechanik wenigstens ein elastisches Federelement auf, welches so ausgebildet ist, dass bei Aufbringen einer Haltekraft zum Festhalten des Wipptisches und gleichzeitiger Fortführung der Antriebsbewegung das Federelement elastisch verformt wird.

Der Wipptisch weist vorzugsweise ein exzentrisch zu der Achse des Wipptisches angeordnetes Kraftaufnahmeelement auf, dessen Auslenkung um den Achsenmittelpunkt ein Schwenken des Wipptisches bewirkt, wobei ferner die Koppelmechanik wenigstens ein Mitnehmerelement aufweist, welches aufgrund der Antriebsbewegung der Antriebseinheit eine Kraft auf das Kraftaufnahmeelement zur Auslenkung des Kraftaufnahmeelementes bewirkt und wobei eine Kraftübertragung von dem Mitnehmerelement auf das Kraftaufnahmeelement an das Federelement gekoppelt ist.

Ferner weist die Inkubationsvorrichtung vorzugsweise eine Haltevorrichtung zum Festhalten wenigstens eines der Wipptische in der festen Position auf.

Die Antriebseinheit ist vorzugsweise unterhalb des Wipptisches angeordnet, wobei die Haltevorrichtung vorzugsweise an ihrer Unterseite ein Eingreifelement mit einer im Wesentlichen konvexen geometrischen Form aufweist, wobei ferner vorzugsweise der Wipptisch an seiner Oberseite eine Eingriffmulde mit einer im Wesentlichen konkaven geometrischen Form aufweist, welche zu der konvexen geometrischen Form des Eingreifelementes korrespondiert, und wobei vorzugsweise ein Heranführen der Haltevorrichtung von oben her in Richtung der Oberseite des Wipptisches der Wipptisch ein Eingreifen des Eingriffelementes in die Eingriffmulde zum Unterbinden der Wippbewegung des Wipptisches in der festen Position bewirkt.

Der Wipptisch weist ferner vorzugsweise ein Metallelement auf, welches mechanisch fest mit dem Wipptisch gekoppelt ist und gemeinsam mit dem Wipptisch die Wippbewegung durchführt, wobei die Inkubationsvorrichtung ferner vorzugsweise einen Elektromagneten sowie eine Steuereinheit aufweist, wobei die Steuereinheit ausgebildet ist, die Haltevorrichtung so anzusteuern, dass die Haltevorrichtung den Wipptisch insbesondere mittels eines Eingreifens des Eingreifelementes in die Eingriffmulde in der festen Position festhält sowie ferner anschließend den Elektromagneten zu aktivieren, so dass eine Magnetkraft zwischen dem Elektromagneten und dem Metallelement den Wipptisch auch dann in der festen Position festhält, wenn die Haltevorrichtung den Wipptisch nicht mehr festhält.

Vorzugsweise wird die Aufnahmevorrichtung vorzugsweise auf einer Oberseite des Wipptisches mit einer nach oben offenen Öffnung ausgebildet, in welche die Inkubationsrinne eingesetzt werden kann.

Die Aufnahmevorrichtung weist vorzugsweise einen Boden auf, auf welchem eine Unterseite der Inkubationsrinne im eingesetzten Zustand zum Aufliegen kommt, und wobei die Aufnahmevorrichtung vorzugsweise ferner einen Klemmmechanismus aufweist, um die Unterseite der Inkubationsrinne auf dem Boden zu halten.

Vorzugsweise weist der Wipptisch ferner eine Heizvorrichtung zum Beheizen des Bodens auf.

Im Folgenden wird die Erfindung anhand spezieller Ausführungsformen ohne Beschränkung des allgemeinen Erfindungsgedankens anhand der Figuren näher erläutert. Dabei zeigen:
Figur 1 eine bevorzugte Ausführungsform einer Inkubationsvorrichtung,
Figur 2 bevorzugte Ausführungsformen von Wipptischen,
Figur 3A bis 3C Details einer bevorzugten Ausführungsform eines Wipptisches,
Figur 4A bis 4C ein Zusammenwirken einer Antriebseinheit mit einem Wipptisch über eine Koppelmechanik gemäß einer bevorzugten Ausführungsform,
Figur 5 eine bevorzugte Ausführungsform einer Antriebseinheit,
Figur 6 ein Teilelement einer bevorzugten Ausführungsform einer Antriebseinheit,
Figuren 7A und 7B eine Verwendung einer Haltevorrichtung,
Figuren 8A und 8B eine Fixierung einer Position eines Wipptisches durch eine Haltevorrichtung,
Figuren 9A und 9B eine bevorzugte Ausführungsform einer Haltevorrichtung,
Figuren 10A bis 10D unterschiedliche Positionen einer Haltevorrichtung und eines Wipptisches bei Verwendung einer Haltevorrichtung,
Figuren 11A bis 11C eine bevorzugte Ausführungsform einer Aufnahmevorrichtung,
Figuren 12A und 12B eine bevorzugte Ausführungsform eines Wipptisches mit einem Metallelement bei einer Verwendung eines Elektromagneten,
Figur 13A und 13B bevorzugte Ausführungsformen einer Wascheinheit,
Figuren 14 eine bevorzugte Ausführungsform einer Inkubationsrinne in einer Aufsicht,
Figuren 15 eine bevorzugte Ausführungsform einer Inkubationsrinne in einer Schnittansicht.

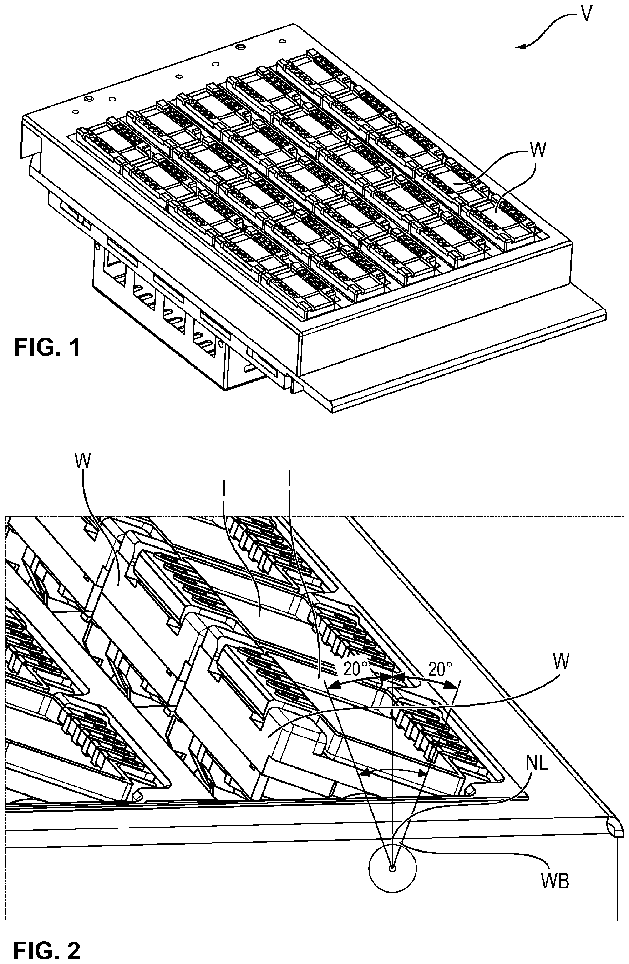

Figur 1 zeigt eine Inkubationsvorrichtung V mit mehreren Wipptischen W. In der Figur 2 ist eine Auslenkung bzw. ein Schwenken der Wipptische W um einen Winkelbereich WB um einen Nulllage NL herum gezeigt. Vorzugsweise beträgt der Winkelbereich plus/minus 20°. Die Wipptische W sind schwenkbar um jeweilige Achsen gelagert. In die Wipptische W sind jeweilige Inkubationsrinnen I eingelegt. Die Inkubationsrinnen sind vorzugsweise ausgeführt wie in der europäischen Patentanmeldung EP 3 085 446 A1 eschrieben.

Vorzugsweise umfasst der vorbestimmte Winkelbereich einen Bereich von -35° bis +35°, vorzugsweise einen Bereich von -25° bis +25°, besonders vorzugsweise einen Bereich von - 20° bis +20° um die Nulllage herum. Der Winkelbereich von -25° bis +25° begünstigt vorteilhafterweise eine Inkubation. Die Winkelbereiche von -25° bis +25° und von -35° bis +35° begünstigen vorteilhafterweise den Flüssigkeitsaustausch in der Inkubationsrinne unter Ausnutzung der Schwerkraft. Des Weiteren wird ein Absaugen begünstigt, ohne dass Inkubationsbereiche in der Inkubationsrinne in einer maximalen Wipplage trocken liegen. In anderen bevorzugten Ausführungsformen umfasst der vorbestimmte Winkelbereich einen Bereich von -90° bis +90° um die Nulllage herum. Der vorbestimmte Winkelbereich ermöglicht das Einstellen des Wippwinkels, vorzugsweise eine Anpassung des Wippwinkels an die Viskosität der Flüssigkeit, um einerseits eine optimale Flüssigkeitsverteilung während der Inkubation zu erreichen und um andererseits zu verhindern, dass Inkubationsbereiche in der Inkubationsrinne in einer maximalen Wipplage trocken liegen. Durch das Einstellen des Wippwinkels wird vorzugsweise ein Auslaufen der Flüssigkeit bzw. ein Überkippen der Inkubationsrinne vermieden.

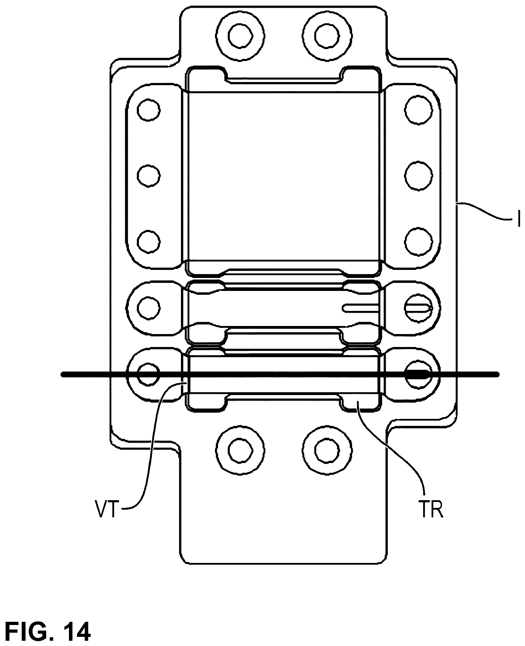

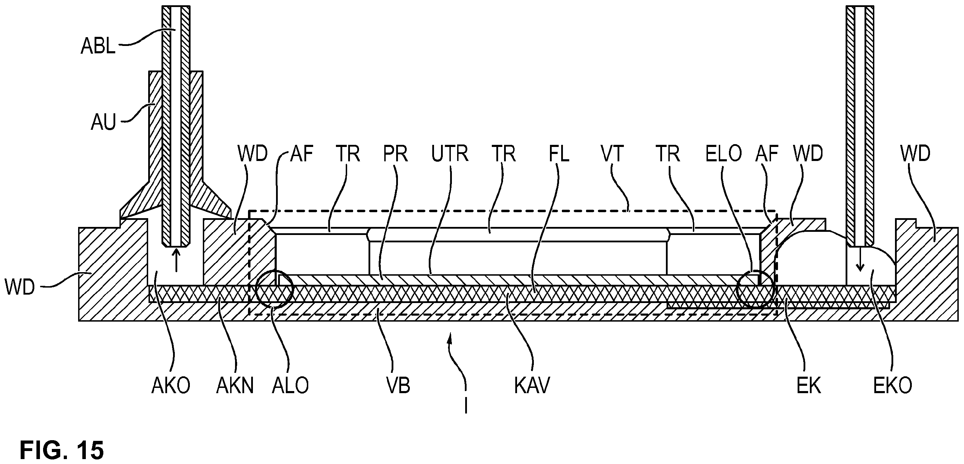

Fig. 14 zeigt eine I in einer bevorzugten Ausführungsform von oben. Vorzugsweise weist die Inkubationsrinne eine Mehrzahl an Teilinkubationsrinnen TR mit einer jeweiligen Vertiefung V auf. Der schwarze Balken markiert die Stelle, an der der in Fig. 15 dargestellte Querschnitt der Inkubationsrinne I liegt.

Fig. 15 zeigt die Inkubationsrinne im Querschnitt an der in Fig. 14 mit dem schwarzen Balken markierten Stelle. Die Inkubationsrinne weist an ihren jeweiligen Längsseiten jeweilige Wände WD auf. An einer Seite der Inkubationsrinne befindet sich ein Auslasskompartiment AKO, das mit der mit einer Flüssigkeit FL befüllbaren Vertiefung VT, gestrichelt gekennzeichnet in Fig. 15, über einen Auslasskanal AKN verbunden ist. Ein in die Inkubationsrinne I eingelegter Träger TR ist weist auf seiner Unterseite eine biologischen Probe PR auf. Bei Einbringen von genügend Flüssigkeit FL ist die Probe PR in die Flüssigkeit FL eingetaucht bzw. zumindest teilweise bedeckt. Durch Anlegen von Unterdruck kann über eine in einer separaten Absaughaube AU befindliche Ableitung ABL Flüssigkeit FL aus einem Auslasskompartiment AKO abgesaugt werden. Über einen Einlasskanal EK kann die Flüssigkeit FL in ein Einlasskompartiment EKO eingeleitet werden. Einlasskanal EK und Auslasskanal AKN münden in der Vertiefung in Gestalt einer Einlasskompartimentöffnung ELO bzw. Auslasskompartimentöffnung ALO. Der Boden des Einlasskompartiments ELO liegt in Fig. 15 vorzugsweise unterhalb der Ebene des Bodens der Vertiefung.

Die Inkubationsrinne I weist somit insbesondere eine von den Wänden WD der Inkubationsrinne IR gebildete Vertiefung VT mit einem Vertiefungsboden VB auf, wobei die Inkubationsrinne I ferner insbesondere wenigstens eine in einen Kanal EK, AKN mündende Öffnung ELO, ALO an einem Längsende der Vertiefung aufweist, bevorzugt in einer Wand WD der Inkubationsrinne I, so dass über den Kanal EK, AKN Flüssigkeit FL in die Vertiefung VT eingebracht oder abgesaugt werden kann. Insbesondere kann in die Inkubationsrinne I ein Träger TR eingelegt werden, welcher bei Einlegen in die Inkubationsrinne I die Probe auf seiner Unterseite UTR, welche dem Vertiefungsboden VB zugewandt ist, aufweist, so dass die Unterseite UTR des Trägers TR, der Vertiefungsboden VB und die Wände WD ein Kompartiment bzw. eine Kavität KAV für die Flüssigkeit FL bilden. Vorzugsweise weist die Inkubationsrinne IR Mittel AF zum Fixieren eines Abstandes zwischen der Unterseite UTR des Trägers TR und dem Vertiefungsboden VB auf, so dass die Unterseite UTR des Trägers TR, der Vertiefungsboden VB und die Wände WD ein Kompartiment bzw. eine Kavität KAV für die Flüssigkeit FL bilden. Diese Mittel sind beispielsweise in Form von Auflageschrägen AF gegeben, auf welchen der Träger TR ein einem oder mehreren Randbereichen der Vertiefung abgelegt werden kann.

In anderen bevorzugten Ausführungsbeispielen umfasst die biologische Probe eine aus einer Gruppe ausgewählte Probe umfassend Gewebe, vorzugsweise Gewebeschnitte oder Gewebebiopsien, z. B. Schnellschnitte, biologische Zellen wie eukaryontische oder prokaryontische Zellen oder Produkte daraus, Viren, aufgereinigte, isolierte oder künstlich hergestellte Moleküle wie Nukleinsäuren, Polypeptide, Lipide oder Kohlenhydrate. Vorzugsweise ist eine biologische Probe menschlichen oder tierischen Ursprungs.

In einem bevorzugten Ausführungsbeispiel der Erfindung weist die Inkubationsrinne einen Auslasskanal mit einer Auslasskanalöffnung zur Entfernung von Flüssigkeit aus der Inkubationsrinne über Absaugen auf, der so ausgestaltet ist, dass der Träger beim Absaugen nicht entfernt oder beschädigt wird. In einem weiteren bevorzugten Ausführungsbeispiel handelt es sich um einen Schlauch, dessen Ende in die Flüssigkeit eingeführt wird. Vorzugsweise handelt es sich um einen in die Inkubationsrinne integrierten Kanal. An den Auslasskanal kann zum Absaugen Druck angelegt werden.

Die Breite des Auslasskanals beträgt vorzugsweise 50 % bis 100 %, besonders vorzugsweise 60 % bis 95 %, noch besonders vorzugsweise 75 % bis 95 % der Breite der Vertiefung der Inkubationsrinne oder des Trägers, vorzugsweise des Trägers.

Die Öffnung des Auslasskanals ist ausgehend von der Ebene des Bodens vertikal so angeordnet, dass wenigstens ein Teil von ihr, vorzugsweise die gesamte Öffnung unterhalb der Oberfläche einer Flüssigkeit liegt, mit der die Inkubationsrinne gefüllt ist, vorzugsweise soweit gefüllt ist, dass der Träger gerade vollständig mit Flüssigkeit bedeckt ist. Vorzugsweise ist die Öffnung am Boden angeordnet. Das ist der Fall, wenn in der Inkubationsrinne befindliche Flüssigkeit, von an Oberflächen anhaftenden Resten abgesehen, vollständig ablaufen kann, wenn die Inkubationsrinne derart geneigt ist, dass die Flüssigkeit in Richtung der Öffnung läuft.

In einem weiteren bevorzugten Ausführungsbeispiel steht der Auslasskanal unter Unterdruck, der vorzugsweise wenigstens derart bemessen ist, dass Flüssigkeit in der Vertiefung abgesaugt wird. Die Förderleistung, mit der die Flüssigkeit abgesaugt wird, kann 0,1 bis 10 I/min, vorzugsweise 0,2 bis 5 I/min, besonders vorzugsweise 0,3 bis 3 I/min betragen. Es können übliche Vorrichtungen zum Absaugen verwendet werden, beispielsweise Membran-, Zahnrad-, Kolben- oder Peristaltikpumpen. Das Absaugen der Flüssigkeit kann kontinuierlich oder diskontinuierlich erfolgen. In diesem bevorzugten Ausführungsbeispiel erfolgt das Absaugen kontinuierlich, d. h. es wird nicht der überwiegende Teil der Flüssigkeit in einem Zug, beispielsweise am Ende des Inkubationsvorganges, abgesaugt, sondern in mehreren Schritten. Vorzugsweise wird bei einem Verfahrensschritt, der das Inkubieren des Trägers in einer Flüssigkeit umfasst, zu wenigstens einem Zeitpunkt, vorzugsweise wenigstens 10, 20, 30, 60, 120, 300, 600 Sekunden, 10, 15, 20 oder 30 Minuten lang gleichzeitig Flüssigkeit an einem Ende der Inkubationsrinne eingelassen und am anderen Ende der Inkubationsrinne abgesaugt. Auf diese Weise kommt der Träger stets nur mit unverbrauchter, frischer Flüssigkeit in Kontakt. Dies beschleunigt die Prozessierung des Trägers bzw. der biologischen Probe. In anderen bevorzugten Ausführungsbeispielen wird der Träger nach dem Einlassen der Flüssigkeit zunächst darin inkubiert, vorzugsweise wenigstens 10, 20, 30, 60, 120, 300 Sekunden, 10, 15 oder 30 Minuten lang, bevor die Flüssigkeit abgesaugt wird.

Die Inkubationsrinne kann mit einer Flüssigkeit gefüllt sein, vorzugsweise einer wässrigen Flüssigkeit, besonders vorzugsweise einem Waschpuffer oder Reagenz zum Prozessieren einer Probe auf dem Träger. Das Volumen der Flüssigkeit wird so bemessen, dass die biologische Probe damit in ausreichendem Kontakt steht. In einem bevorzugten Ausführungsbeispiel ist das Volumen der Flüssigkeit in der Inkubationsrinne so bemessen, dass sie Träger und Probe darauf in horizontaler Lage vollständig bedeckt. In anderen bevorzugten Ausführungsbeispielen ist es so bemessen, dass die Flüssigkeit eine Probe vollständig bedeckt, die an der Seite des Trägers angeordnet ist, welche dem Boden zugewandt ist. Alternativ ist es so bemessen, dass es die Probe nicht andauernd, aber regelmäßig vollständig benetzt, wenn die Inkubationsrinne beim Inkubieren geschwenkt bzw. gewippt wird. Während bei leicht verfügbaren Lösungen wie unspezifischen Waschpuffern, beispielsweise PBS, oder Lösungen zum Entwickeln eines Signals ein Überschuss eingesetzt werden kann, beschränkt sich der Anwender bei anderen Lösungen wie schwierig zu gewinnenden, nur in geringen Volumina verfügbaren Reagenzien, beispielsweise Antikörpern, insbesondere primären Antikörpern, auf das absolut notwendige Mindestvolumen.

Optional weist die Inkubationsrinne einen Einlasskanal auf. Dabei handelt es sich um ein abgeschlossenes Mittel zum Zufluss von Flüssigkeit, das nicht druckabschließend ausgeführt sein muss. Der Zufluss kann direkt in die Inkubationsrinne erfolgen.

Das Inkubationsmodul bzw. die Vielzahl von Inkubationsrinnen oder die Vielzahl der Wipptische kann vorzugsweise derart bewegt werden, dass die Flüssigkeit durchmischt und ihre Exposition gegenüber dem jeweiligen Träger gefördert wird, beispielsweise durch Schwenken bzw. Wippen, Vibrieren, Schütteln oder dergleichen. In bevorzugten Ausführungsbeispielen ist die Inkubationsrinne über die Achse schwenkbar, so dass sich die Flüssigkeit in Richtung des tiefer liegenden Längsendes der Inkubationsrinne verschiebt. Im geschwenkten Zustand, besonders wenn die Auslassöffnung sich am Längsende befindet und die Auslasskanalöffnung sich an deren Boden befindet, ist das Entnehmen der Flüssigkeit besonders einfach und effizient. Beim Schwenken bildet die Inkubationsrinne mit der Grundfläche vorzugsweise einen Winkel von 1° bis 45°, besonders vorzugsweise 2,5° bis 30°, noch besonders vorzugsweise 7,5° bis 25° aus.

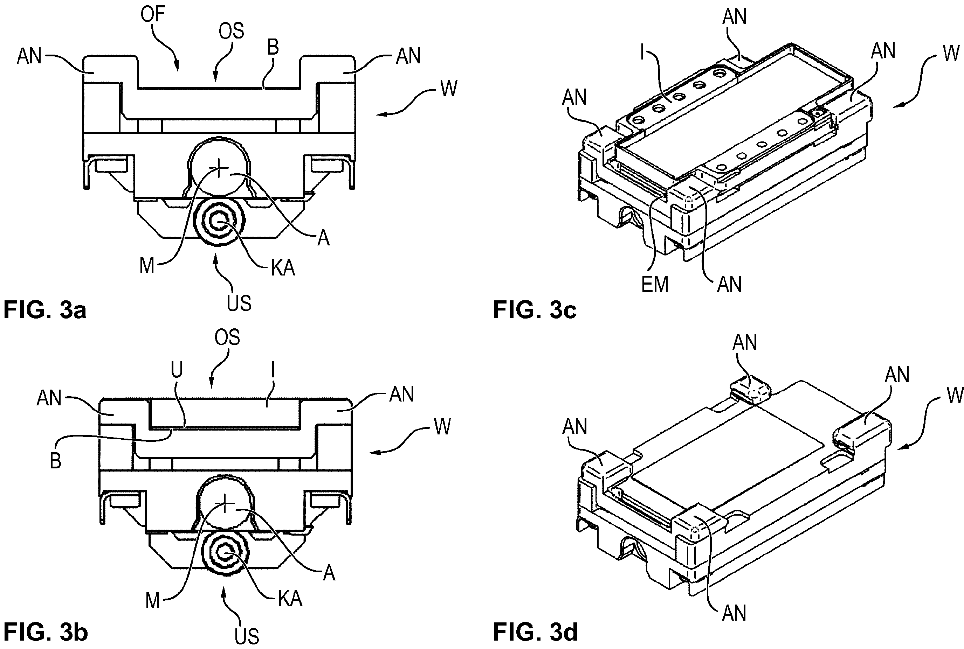

Die Figur 3A zeigt einen Wipptisch W in einer Seitenansicht ohne eine eingelegte Inkubationsrinne. Die Figur 3B zeigt einen Wipptisch W in einer Seitenansicht mit einer eingelegten Inkubationsrinne I. Der Wipptisch W ist um eine Achse A mit einem Mittelpunkt M schwenkbar gelagert. Eingelegt in eine Aufnahmevorrichtung AN des Wipptisches W ist in der Figur 3B eine Inkubationsrinne I, welche eine Unterseite U aufweist, die auf einem Boden B einer Öffnung OF der Aufnahmevorrichtung AN aufliegt. Dargestellt ist auch die Oberseite OS des Wipptisches sowie dessen Unterseite US. Über ein Kraftaufnahmeelement KA kann der Wipptisch W um seine Achse A geschwenkt werden. Die Achse A ist insbesondere eine Längsachse, welche entlang der größeren Ausdehnungsrichtung des Wipptisches verläuft. Über die Achse A kann ein Querschwenken des Wipptisches vorgenommen werden.

Die Figur 3C zeigt in einer Seitenansicht von schräg oben noch einmal den Wipptisch W mit eingelegter Inkubationsrinne I. Die Inkubationsrinne I liegt in der Aufnahmevorrichtung AN ein. Ferner dargestellt ist eine Eingriffmulde EM, auf welche später noch genauer eingegangen wird. Die Figur 3D zeigt in der Seitenansicht von schräg oben noch einmal den Wipptisch W ohne Inkubationsrinne.

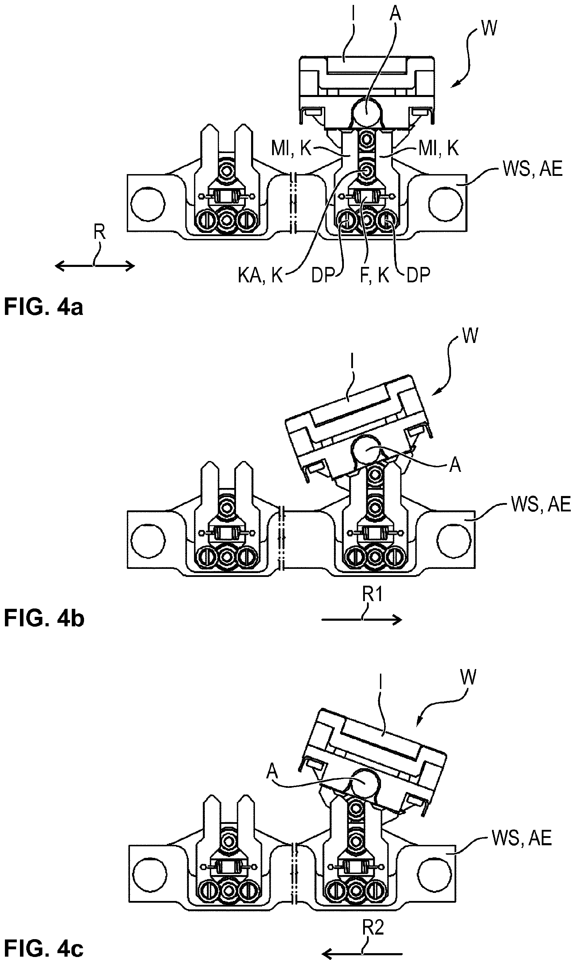

Die Figur 4A zeigt den Wipptisch W zusammen mit einem Wippschieber WS, welcher Teil einer Antriebseinheit AE sein kann. Der Wippschieber WS kann eine Antriebsbewegung in lateraler Ebene, dargestellt als die Richtung bzw. Ebene R, ausführen, um mittels einer Koppeleinheit K, welche aus mehreren Komponenten bestehen kann, auf das Kraftaufnahmeelement KA zu wirken, um eine Wippbewegung des Wipptisches W herbeizuführen. Die Antriebseinheit erzeugt somit vorzugsweise eine Lateralbewegung als Antriebsbewegung. Die Lateralbewegung der Antriebseinheit AE wird mittels der Koppelmechanik K in eine Wippbewegung des Wipptisches W umgewandelt. Das Kraftaufnahmeelement KA ist exzentrisch zu der Achse A des Wipptisches W angeordnet, sodass eine Auslenkung des Kraftaufnahmeelementes KA um den Achsenmittelpunkt der Achse A ein Schwenken des Wipptisches bewirkt.

Die Koppelmechanik K besteht in diesem Ausführungsbeispiel aus wenigstens einem Mitnehmerelement MI, dem Kraftaufnahmeelement KA sowie wenigstens einem Federelement F. Das Kraftaufnahmeelement KA an dem Wipptisch W kann als Teil der Koppelmechanik K angesehen werden. Wird das Kraftaufnahmeelement KA durch Kraftübertragung einer Kraft von einem der Mitnehmerelemente MI in der Richtung bzw. der Ebene R ausgelenkt, so kommt es zu einer Wippbewegung des Wipptisches W, wie in den Figuren 4B und 4C für die spezifischen Richtungen R1 bzw. R2 dargestellt. In der Nulllage liegt die Inkubationsrinne in dem Wipptisch in einer ebenen Lage gegenüber der Erdoberfläche.

Mit anderen Worten: Die Koppelmechanik K weist wenigstens ein Mitnehmerelement MI auf, welches aufgrund der Antriebsbewegung der Antriebseinheit AE eine Kraft auf das Kraftaufnahmeelemente KA zur Auslenkung des Kraftaufnahmeelementes bewirkt, wobei eine Kraftübertragung von dem Mitnehmerelement MI auf das Kraftaufnahmeelement KA an das Federelement F gekoppelt ist.

Die Mitnehmerelemente MI sind an sogenannten Drehpunkten DP drehbar gelagert. Das Federelement F ist so ausgestaltet, dass ohne Festhalten des Wipptisches W die Antriebsbewegung der Antriebseinheit AE bzw. des Wippschiebers WS die Wippbwegung des Wipptisches W um den Winkelbereich bewirkt. Erfolgt kein Festhalten des Wipptisches durch eine Haltekraft, so wird bei Durchführung bzw. Fortführung der Antriebsbewegung zum Bewirken der Wippbewegung das Federelement F im Wesentlichen nicht verformt. Hierdurch wird erreicht, dass eine Antriebskopplung zwischen der Antriebseinheit AE bzw. dem Wippschieber WS und dem Wipptisch W so ausgestaltet ist, dass sich kein spürbares Mechanikspiel zwischen der Antriebseinheit AE und dem Wipptisch W ergibt. Es könnte sonst bei einem merklichen Mechanikspiel zu unerwünschten Schwingungen bzw. unerwünschten Bewegungen des Wipptisches W kommen. Solch unerwünschte Bewegungen könnten ein kontrolliertes Fließen der Flüssigkeit in der Inkubationsrinne stören.

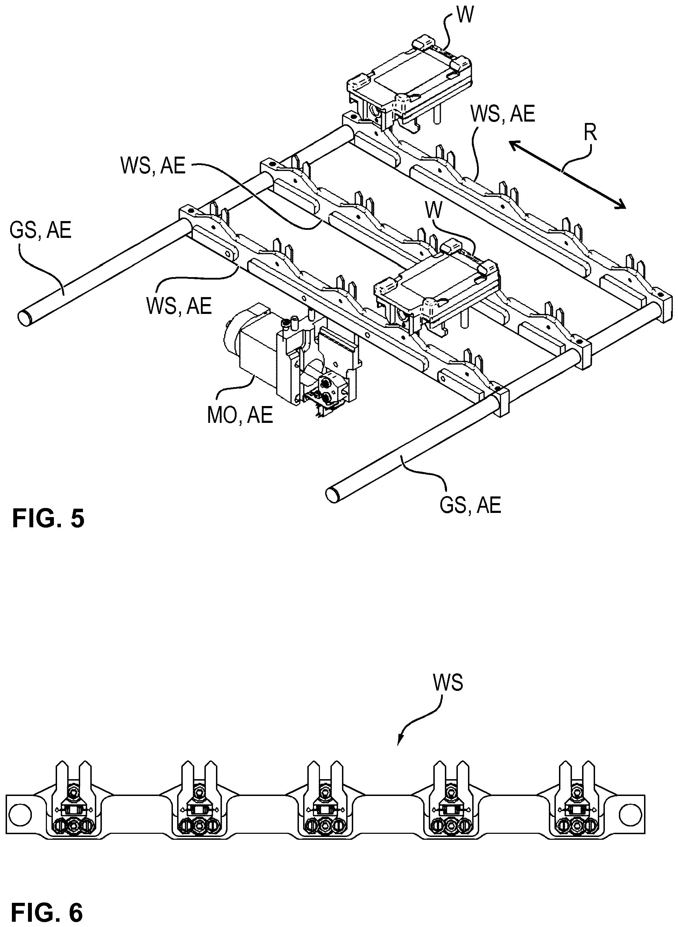

Figur 5 zeigt eine bevorzugte Ausführungsform einer Antriebseinheit AE, bei welcher ein Motor M, vorzugsweise ein Schrittmotor, mit wenigsten einem Wippschieber WS als Teil der Antriebseinheit AE gekoppelt ist. Vorzugsweise sind mehrere Wippschieber WS mit dem Motor MO gekoppelt, insbesondere über ein Gestänge GS. Der Motor unterzieht so die Wippschieber WS der Antriebsbewegung in der Ebene bzw. Richtung R. Hierdurch werden dann also mehrere Wipptische W den entsprechenden Wippbewegungen unterzogen. Die einzelnen Achsen der einzelnen Wipptische W können vorzugsweise auch als gemeinsame Drehachsen ausgeführt werden, wenn mehrere Wipptische hintereinander angeordnet sind, wie in der Figur 2 dargestellt.

Figur 6 zeigt eine bevorzugte Ausführungsform eines Wippschiebers WS zum Schwenken bzw. Wippen mehrerer Wipptische nebeneinander.

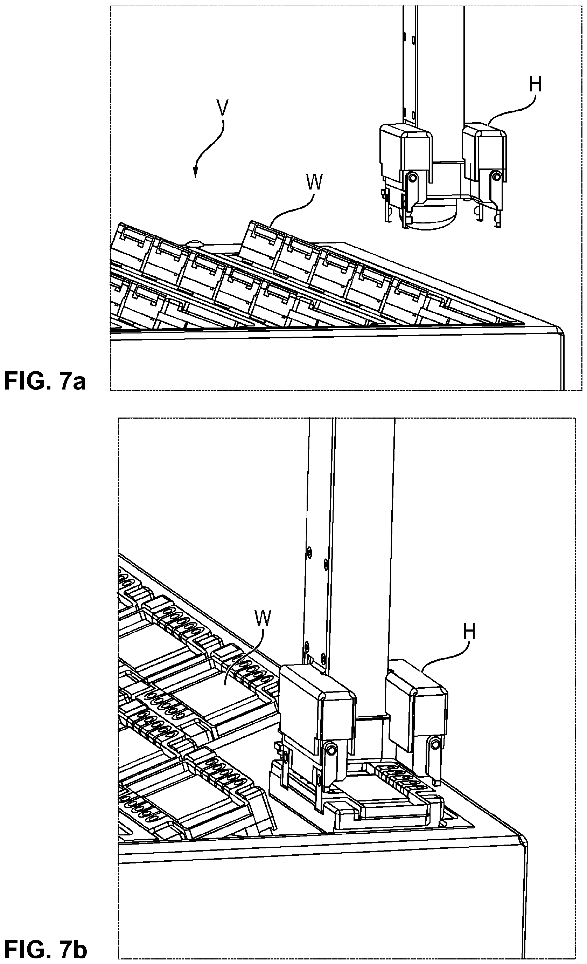

Die Figur 7A zeigt ein Heranführen einer Haltevorrichtung H an einen Wipptisch.

In der Figur 7B ist gezeigt, wie die Haltevorrichtung H einen einzelnen Wipptisch W festhält, während die anderen Wipptische weiterhin der Wippbewegung aufgrund der Antriebsbewegung unterzogen werden.

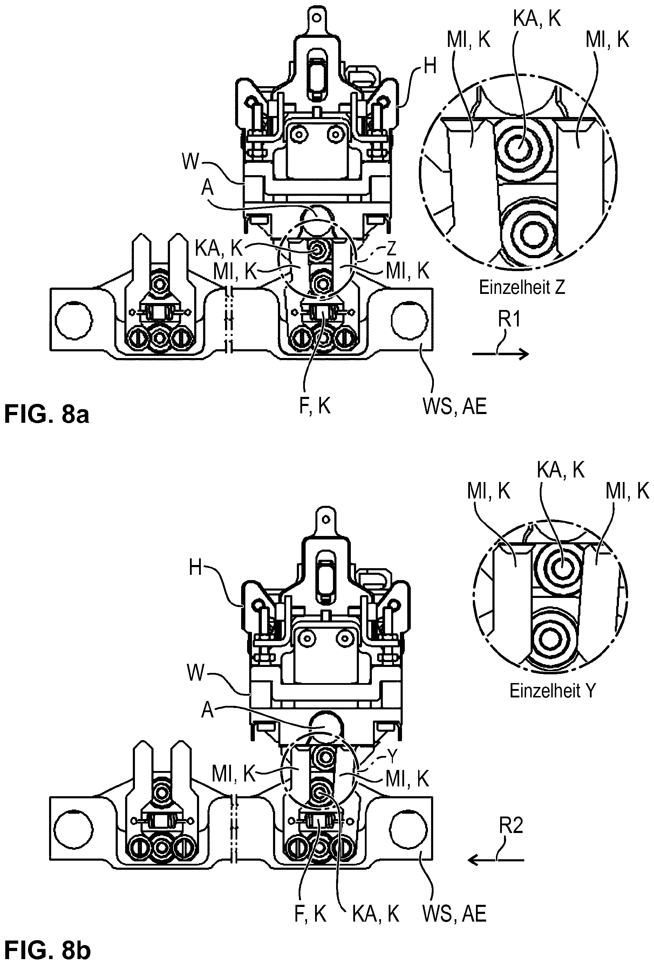

Die Figur 8A zeigt in einer Seitenansicht nochmals die Haltevorrichtung H, welche den Wipptisch W festhält, sodass der Wipptisch W nicht mehr um die Achse A rotieren kann. Deatils sind hierzu insbesondere in der Einzelheit Z dargestellt. Obwohl der Wippschieber WS weiterhin die Antriebsbewegung in der Richtung R1 durchführt, bewirkt der Mitnehmer MI auf der linken Seite trotz Anliegen an dem Kraftaufnahmeelement KA keine Auslenkung bzw. Schwenkung des Wipptisches mehr. Dieses wird dadurch erreicht, dass die Koppelmechanik K derart elastisch federnd eine Kopplung zwischen dem Wipptisch W und der Antriebseinheit AE bzw. dem Wippschieber WS bewirkt, dass bei Festhalten des Wipptisches W der Wipptisch W trotz Fortführung der Antriebsbewegung in einer festen Position stehen bleiben kann.

Das Federelement F ist so ausgebildet, dass es bei Aufbringen einer Haltekraft zum Festhalten des Wipptisches, insbesondere in der Nulllage, und gleichzeitiger Fortführung der Antriebsbewegung lediglich elastisch aber nicht plastisch verformt wird. In dem Fall, dass keine Haltekraft zum Festhalten des Wipptisches aufgebracht wird, wird das Federelement F bei Fortführung der Antriebsbewegung zum Bewirken der Wippbewegung im Wesentlichen nicht verformt. Hierdurch wird es also ermöglicht, den Wipptisch festzuhalten, vorzugsweise durch eine Haltevorrichtung H, um dann eine Inkubationsrinne in den Wipptisch W einzulegen oder herauszunehmen. Es wird ferner möglich, während des Festhaltens ein Absaugen von Flüssigkeit in der Inkubationsrinne vorzunehmen oder aber Flüssigkeit in die Inkubationsrinne zu pipettieren. Die dynamischen Eigenschaften der Wippbewegung des Wipptisches W für den weiteren Betrieb bzw. Gebrauch werden jedoch durch das Festhalten des Wipptisches W nicht beeinflusst, da das Federelement F in der Weise wie zuvor ausgeführt ausgebildet ist, sodass die mechanischen Eigenschaften der mechanischen Kopplung zwischen der Antriebseinheit und dem Wipptisch aufgrund des Festhalten des Wipptisches nicht verändert werden. Das Festhalten kann als ein Unterbinden der Wippbewegung ohne Veränderung der mechanischen Eigenschaften der Koppeleinheit zum schwenken des Wipptisches für einen späteren Betrieb angesehen werden.

Die Figur 8B zeigt den analogen Effekt zu jenem, welcher in der Figur 8A beschrieben wurde, in dem Fall, dass die Bewegung des Wippschiebers WS in die zu der Richtung R1 aus Figur 8a entgegengesetzte Richtung R2 erfolgt. Es kommt zu einer Auslenkung des rechtsseitigen Mitnehmerelementes MI aufgrund der elastischen Feder F. Details sind hierbei in der Einzelheit Y gezeigt.

Durch die erfindungsgemäße Inkubationsvorrichtung ist es möglich, den Wipptisch in einer beliebigen Position innerhalb des Winkelbereiches festzuhalten, ohne die Eigenschaften der mechanischen Kopplung zwischen der Antriebseinheit und dem Wipptisch für einen späteren Betrieb bzw. zu späteren Zeitpunkten zu beeinträchtigen, da die Koppelmechanik elastisch federnd ausgebildet ist.

Das Festhalten des Wipptisches kann nicht nur in der Weise erfolgen, wie in der Figur 8A oder Figur 8B gezeigt, sondern dies kann auch in einer sogenannten Schräglage des Wipptisches erfolgen, um ein Fließen von Flüssigkeit innerhalb der Inkubationsrinne zu einer der Seiten in der Inkubationsrinne zu erzwingen. Das Festhalten des Wipptisches ist insbesondere ein Festhalten entgegen der Wippbewegung. Insbesondere kann dies durch Aufbringen einer Haltekraft von außen erfolgen. Der Wipptisch bleibt bei Festhalten des Wipptisches trotz weiterhin ausgeführter Antriebsbewegung der Antriebseinheit in fester Position stehen.

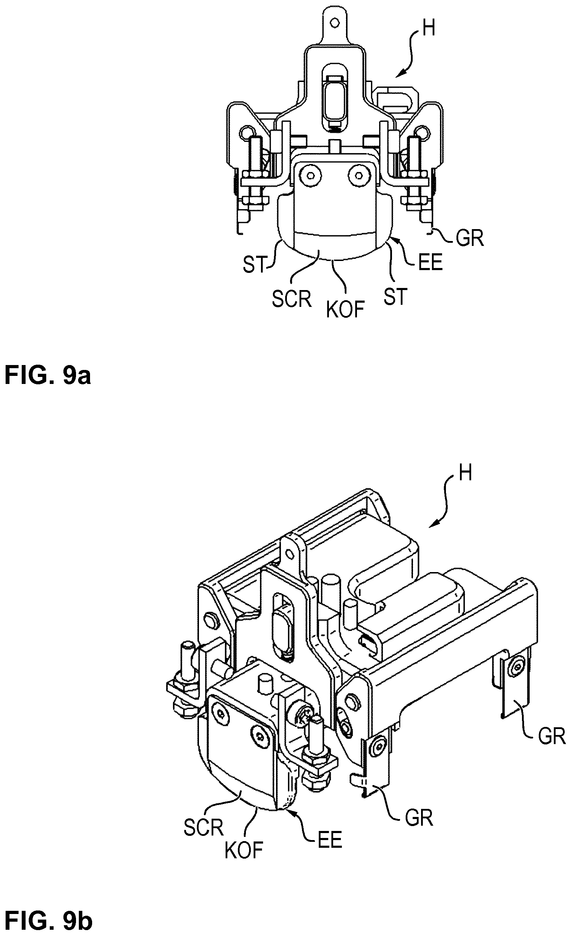

Die Figur 9A zeigt eine bevorzugte Ausführungsform einer Haltevorrichtung, welche an ihrer Unterseite ein Eingriffelement EE aufweist, welches eine im Wesentlichen konvexe geometrische Form KOF aufweist. Die Haltevorrichtung H weist ferner ein Greifelement GR auf, welches zu den Seiten hin verschiebbar sind, um eine Inkubationsrinne während eines Transportvorganges zu greifen oder aber um eine Inkubationsrinne aus einem Wipptisch herauszunehmen bzw. herauszugreifen. Mittels der Haltevorrichtung H kann also vorzugsweise unter Verwendung der Greifelemente GR eine Inkubationsrinne hin zu einem Wipptisch transportiert werden. Ferner kann nach Herausfahren der Greifelemente GR zu den Seiten hin die Inkubationsrinne in einem Wipptisch eingelegt werden.

Die Figur 9B zeigt die Haltevorrichtung noch einmal aus einer weiteren Perspektive, in welcher auch eine Schräge SCR des Eingrifselementes EE deutlich bzw. sichtbar wird. Das Eingriffelement EE kann bei Heranführen der Haltevorrichtung H an einen Wipptisch W dann in eine Eingriffmulde EM, wie in der Figur 3B gezeigt, eingreifen. Die Eingriffmulde EM weist dann eine im Wesentlichen konkave geometrische Form auf, welche zu der komplexen geometrischen Form des Eingriffelementes EE korrespondiert.

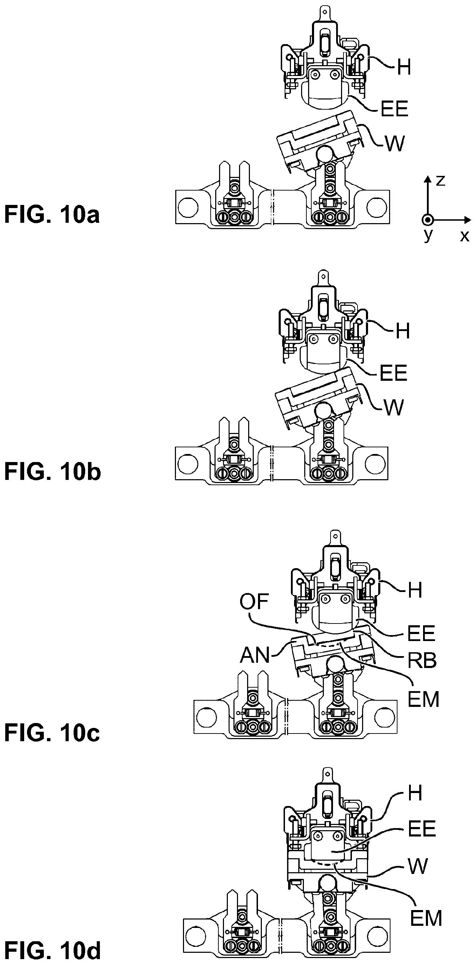

Die Figuren 10A bist 10D zeigen die Haltevorrichtung H in unterschiedlichen Positionen gegenüber einem Wipptisch W, welcher zumindest teilweise noch aufgrund einer Antriebsbewegung der Antriebseinheit eine Wippbewegung durchführt. Der Wipptisch W vollzieht die Wippbewegung in einer XZ-Ebene, wie aus der Figur 10A ersichtlich, wobei die Drehachse, um welche der Wipptisch W geschwenkt wird bzw. wirkt, in der Y-Ebene, senkrecht zur Bildebene der Darstellung, verläuft. Die Haltevorrichtung H wird in Z-Richtung an die Oberseite des Wipptisches angeführt. Hierbei greift das Eingriffelement EE in die Eingriffmulde EM, in der Figur 10C und 10D gestrichelt eingezeichnet, ein. Durch die gestrichelte Darstellung der Eingriffmulde EM in den Figuren 10C und 10D in Zusammenschau mit der Darstellung der oberen Öffnung der Eingriffmulde EM aus der Figur 3B wird für den Fachmann ersichtlich, in welcher Weise die Eingriffmulde EM auszuführen ist. Das Eingreifen des Eingriffelementes EE in die Eingriffmulde EM unterbindet die Wippbewegung des Wipptisches W in der festen Position, wie in der Figur 10D gezeigt.

Wie aus der Figur 9A sowie 9B ersichtlich wird, ist das Eingriffelement EE an seinen äußeren Seiten ST abgerundet. Hierdurch wird bewirkt, dass bei Heranführen der Haltevorrichtung H an den Wipptisch W unabhängig von einer tatsächlichen Lage des Wipptisches W das Eingriffelement EE auch in einem Randbereich RB der Aufnahmevorrichtung AN in deren Öffnung OF eingreifen kann, ohne eine mechanische Arretierung bzw. mechanische Blockierung in einem der Zwischenzustände aus der Figur 10B oder 10C während des Heranführens der Haltevorrichtung H an den Wipptisch W zu bewirken.

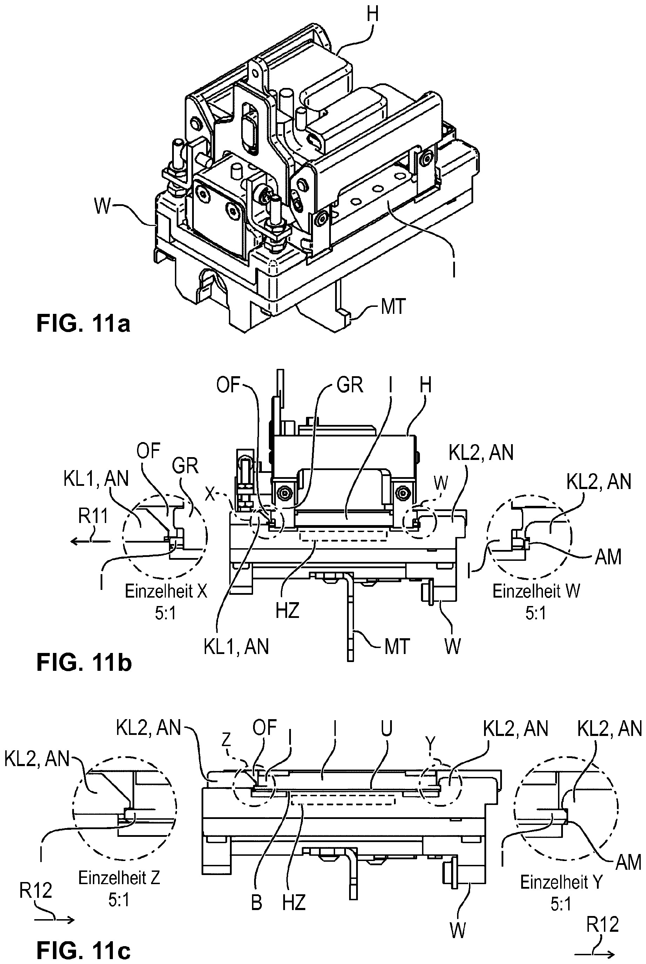

Die Figur 11A zeigt eine Haltevorrichtung H mit einem Wipptisch W in einer weiteren Ausführungsform, wobei in den Wipptisch W eine Inkubationsrinne I eingelegt ist. Unterhalb des Wipptisches W befindet sich ein an dem Wipptisch W befestigtes Metallelement auf der Unterseite des Wipptisches.

Die Figur 11B zeigt eine auf einer Oberseite des Wipptisches W befindliche Öffnung OF der Aufnahmevorrichtung AN. Wie zuvor unter Bezug auf die Figur 3A ausgeführt, weist die Aufnahmevorrichtung AN einen Boden B auf, auf welchem eine Unterseite U der Inkubationsrinne im eingesetzten Zustand zum Aufliegen kommt.

Vorzugsweise weist der Wipptisch eine Heizvorrichtung HZ zum Heizen einer Inkubationsrinne I auf.

Die Aufnahmevorrichtung AN weist ferner einen Klemmmechanismus auf, welcher in dieser Ausführungsform durch zwei Klemmmechanismuselemente KL1, KL2 gegeben ist. Der Klemmmechanismus KL1, KL2 bewirkt ein Halten der Unterseite der Inkubationsrinne I auf dem Boden B des Wipptisches W. Hierdurch wird also eine feste mechanische Fixierung bzw. Halterung einer Inkubationsrinne auf dem Wipptisch erreicht, sodass die Inkubationsrinne I sicher in dem Wipptisch W während einer Durchführung einer Wippbewegung gelagert ist.

Jener Teil des Klemmmechanismus KL1, welcher in der Einzelheit X genauer gezeigt ist, ist verschiebbar in der Weise, dass er in eine Richtung R11 nach außen hin verschoben werden kann, sodass dann ein Vorsprung der Inkubationsrinne I freigelegt wird. In der Einzelheit X ist auch noch ein Teil GR des Enzkreiselementes zu sehen.

Das Klemmmechanismuselement KL1 kann auch entgegen der Richtung R11 wieder zurück verschoben werden, vorzugsweise durch eine Federkraft. Dies ist in der Figur 11C in der Einzelheit Z dargestellt. Es hält also das Klemmmechanismuselement KL1 die Inkubationsrinne I in diesem Bereich, wie als Einzelheit Z in der Figur 11C dargestellt, fest.

Ferner ist ein Klemmmechanismuselement KL2 in der Einzelheit W der Figur 11B dargestellt, welches eine Ausnehmung AN aufweist. Es wird bei Verschieben der Inkubationsrinne I in der Richtung R12 ein Eingreifen der Inkubationsrinne I in eine Ausnehmung AN des Klemmmechanismuselementes KL2 herbeigeführt, wie auch in der Figur 11C in der Einzelheit Y dargestellt,.

Der Klemmmechanismus KL1, KL2 bewirkt nicht nur ein festes Fixieren der Inkubationsrinne I auf dem Wipptisch W zum Festhalten der Inkubationsrinne I während einer Durchführung der Wippbewegung durch den Wipptisch W. Der Klemmechanismus KL1, KL2 kann ferner durch das Halten der Unterseite der Inkubationsrinne auf dem Boden der Öffnung der Aufnahmevorrichtung bewirken, dass ein thermischer Übergang bzw. eine thermische Kontaktierung zwischen der Heizvorrichtung HZ des Wipptisches W und der Inkubationsrinne I maximiert wird bzw. gewährleistet wird. Ein Aufheizen von Flüssigkeit kann im Zuge einer pathohistochemischen Prozessierung einer Probe notwendig sein. Im Zuge eines Heizens kann es zu einem Durchbiegen der Inkubationsrinne I kommen, sodass diese nicht mehr vollflächig mit ihrer Unterseite U auf dem Boden B aufliegt sondern sich wölbt, sodass der thermische Übergang bzw. die thermische Kontaktierung reduziert wird. Dadurch, dass der Klemmmechanismus KL1, KL2 die Unterseite U des Bodens der Inkubationsrinne I auf dem Boden B festhält, wird der thermische Übergang bzw. die thermische Kontaktierung verbessert.

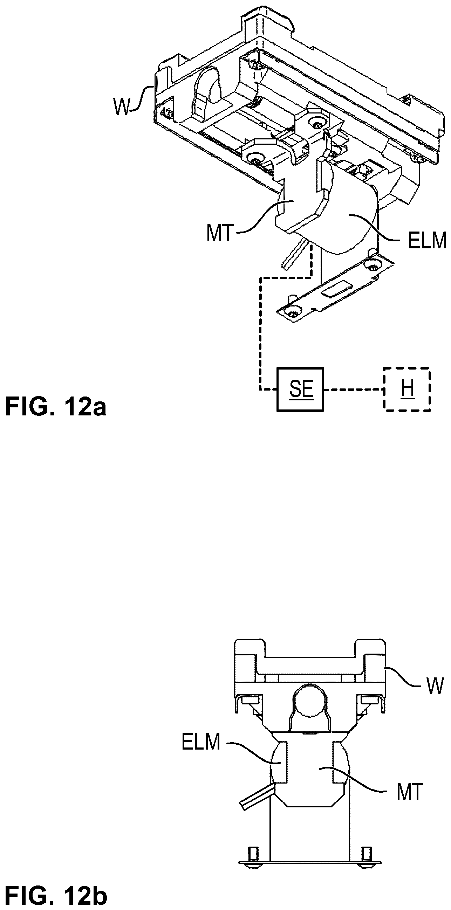

Die Figur 12A zeigt im Detail ein Metallelement MT, welches mechanisch fest mit dem Wipptisch W gekoppelt ist und mit dem Wipptisch die Wippbewegung durchführt. Ferner gezeigt ist ein Elektromagnet ELM, welcher vorzugsweise über eine schematisch dargestellte Steuereinheit SE angesteuert bzw. aktiviert und deaktiviert werden kann. Die Steuereinheit ist ferner ausgebildet, die Haltevorrichtung anzusteuern, wie schematisch in Figur 12A illustriert.

Die Steuereinheit SE ist so ausgebildet, dass die Haltevorrichtung H in der Weise angesteuert wird, dass diese den Wipptisch vorzugsweise mittels eines Eingreifens des Eingriffelement ist die Eingriffmulde in der festen Position festhält. Die Steuereinheit ist ferner ausgebildet, anschließend den Elektromagneten zu aktivieren, sodass eine Magnetkraft zwischen dem Elektromagneten und die Metallelement den Wipptisch auch dann in der festen Position festhält, wenn die Haltevorrichtung des Wipptisches nicht mehr festhält. Hierdurch wird erreicht, dass zunächst durch eine Haltevorrichtung H der Wipptisch W in eine feste Position gebracht werden kann, welche dann jedoch dem späteren Zeitpunkt allein durch den Elektromagnet ELM bewirkt werden kann, sodass die Haltevorrichtung H sich wieder von dem Wipptisch W entfernen kann. Hierdurch kann dann also zu einem späteren Zeitpunkt einer Pipettiereinheit hin an die Inkubationsrinne gebracht werden, ohne dass die Haltevorrichtung H noch den Wipptisch W festhalten muss, sodass also mehr Platz im Bereich der Inkubationsrinne zum Pipettieren vorhanden ist.

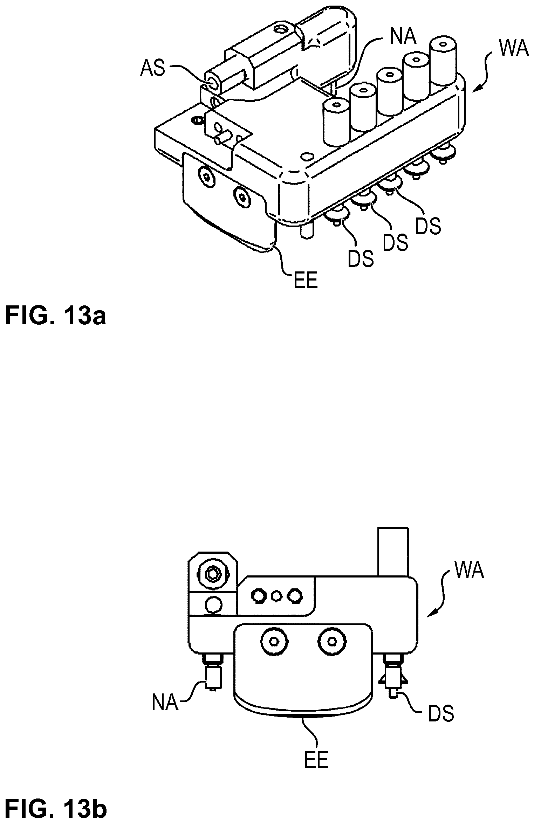

Die Figur 13A zeigt eine vorbevorzugte Einheit einer Wascheinheit WA, welche über einzelne Saugvorrichtungen DS, vorzugsweise Düsen oder Ansaugdüsen DS, an der Inkubationsrinne Flüssigkeit absaugen kann. Vorzugsweise zeigt die Wascheinheit WA eine eigene Eingriffseinheit EE auf. Über einzelne Nadeln NA kann über eine Anschlusseinheit AS dann mittels eines Pipettierens Flüssigkeit hin zu einer Inkubationsrinne gebracht werden.

Figur 13B zeigt die Wascheinheit WA in einer Vorderansicht.

## Patentansprüche

1. Inkubationsvorrichtung (V),
- aufweisend eine Mehrzahl an Wipptischen (W), welche jeweils um eine jeweilige Achse (A) schwenkbar gelagert sind, wobei ein jeweiliger Wipptisch (W) eine jeweilige Aufnahmevorrichtung (AN) für eine mechanisch reversible Aufnahme einer jeweiligen Inkubationsrinne (I) aufweist,
- ferner aufweisend eine gemeinsame Antriebseinheit (AE) zum gemeinsamen Bewirken jeweiliger Wippbewegungen der jeweiligen Wipptische, insbesondere aus einer Nulllage (NL) heraus, um einen bestimmten Winkelbereich (WB) aufgrund einer Antriebsbewegung der Antriebseinheit (AE),
wobei jeder der jeweiligen Wipptische (W) mit der Antriebseinheit (AE) über eine jeweilige Koppelmechanik (K) gekoppelt ist,
und wobei jede der jeweiligen Koppelmechaniken (K) den jeweiligen Wipptisch (W) mit der Antriebseinheit (AE) derart elastisch federnd koppelt, dass bei Festhalten des Wipptisches (W) der Wipptisch (W) trotz Fortführung der Antriebsbewegung in einer festen Position stehen bleibt, ferner jedoch nach Unterlassen des Festhaltens des Wipptisches (W) der Wipptisch (W) wieder aufgrund der Antriebsbewegung die Wippbewegung um den bestimmten Winkelbereich (WB) durchführt.

2. Inkubationsvorrichtung nach Anspruch 1,
wobei die Koppelmechanik (K) wenigstens ein elastisches Federelement (F) aufweist, welches so ausgebildet ist, dass bei Aufbringen einer Haltekraft zum Festhalten des Wipptisches (W) und gleichzeitiger Fortführung der Antriebsbewegung das Federelement (F) elastisch verformt wird.

3. Inkubationsvorrichtung nach Anspruch 2,
wobei der Wipptisch (W) ein exzentrisch zu der Achse (A) des Wipptisches (W) angeordnetes Kraftaufnahmeelement (KA) aufweist, dessen Auslenkung um den Achsenmittelpunkt (M) ein Schwenken des Wipptisches (W) bewirkt,
wobei ferner die Koppelmechanik (K) wenigstens ein Mitnehmerelement (MI) aufweist, welches aufgrund der Antriebsbewegung der Antriebseinheit (AE) eine Kraft auf das Kraftaufnahmeelement (KA) zur Auslenkung des Kraftaufnahmeelementes (KA) bewirkt
und wobei eine Kraftübertragung von dem Mitnehmerelement (MI) auf das Kraftaufnahmeelement (KA) an das Federelement (F) gekoppelt ist.

4. Inkubationsvorrichtung nach Anspruch 1,
ferner aufweisend eine Haltevorrichtung (H) zum Festhalten wenigstens eines der Wipptische (W) in der festen Position.

5. Inkubationsvorrichtung nach Anspruch 4,
wobei die Antriebseinheit (AE) unterhalb des Wipptisches (W) angeordnet ist, wobei die Haltevorrichtung (H) an ihrer Unterseite ein Eingreifelement (EE) mit einer im Wesentlichen konvexen geometrischen Form (KOF) aufweist,
wobei ferner der Wipptisch (W) an seiner Oberseite eine Eingriffmulde (EM) mit einer im Wesentlichen konkaven geometrischen Form (KOF) aufweist, welche zu der konvexen geometrischen Form des Eingreifelementes (EE) korrespondiert,
und wobei ein Heranführen der Haltevorrichtung (H) von oben her in Richtung der Oberseite des Wipptisches (W) ein Eingreifen des Eingriffelementes (EE) in die Eingriffmulde (EM) zum Unterbinden der Wippbewegung des Wipptisches (W) in der festen Position bewirkt.

6. Inkubationsvorrichtung nach Anspruch 2,
wobei der Wipptisch (W) ferner ein Metallelement (MT) aufweist, welches mechanisch fest mit dem Wipptisch (W) gekoppelt ist und gemeinsam mit dem Wipptisch (W) die Wippbewegung durchführt,
wobei die Inkubationsvorrichtung (V) ferner einen Elektromagneten (ELM) sowie eine Steuereinheit (SE) aufweist,
wobei die Steuereinheit (SE) ausgebildet ist, die Haltevorrichtung (H) so anzusteuern, dass die Haltevorrichtung (H) den Wipptisch (W) insbesondere mittels eines Eingreifens des Eingreifelementes (EE) in die Eingriffmulde (EM) in der festen Position festhält sowie ferner anschließend den Elektromagneten (ELM) zu aktivieren, so dass eine Magnetkraft zwischen dem Elektromagneten (ELM) und dem Metallelement (MT) den Wipptisch (W) auch dann in der festen Position festhält, wenn die Haltevorrichtung (H) den Wipptisch (W) nicht mehr festhält.

7. Inkubationsvorrichtung nach Anspruch 1,
wobei die Aufnahmevorrichtung (AN) auf einer Oberseite des Wipptisches (W) mit einer nach oben offenen Öffnung (OF) ausgebildet wird, in welche die Inkubationsrinne (I) eingesetzt werden kann.

8. Inkubationsvorrichtung nach Anspruch 7,
wobei die Aufnahmevorrichtung (AN) einen Boden (B) aufweist, auf welchem eine Unterseite (U) der Inkubationsrinne (I) im eingesetzten Zustand zum Aufliegen kommt,
und wobei die Aufnahmevorrichtung (AN) ferner einen Klemmmechanismus (KL1, KL2) aufweist, um die Unterseite (U) der Inkubationsrinne (I) auf dem Boden (B) zu halten.

9. Inkubationsvorrichtung nach Anspruch 8,
wobei der Wipptisch (W) ferner einer Heizvorrichtung (HZ) zum Beheizen des Bodens (B) aufweist.

10. System
aufweisend eine Inkubationsvorrichtung (V) nach einem der Ansprüche 1 bis 9 sowie wenigstens eine in einen Wipptisch (W) einlegbare Inkubationsrinne (I).

11. System nach Anspruch 10,
wobei die Inkubationsrinne (I) eine von den Wänden (WD) der Inkubationsrinne (I) gebildete Vertiefung (VT) mit einem Vertiefungsboden (VB) aufweist,
wobei die Inkubationsrinne (I) wenigstens eine in einen Kanal (AKN, EK) mündende Öffnung (ALO, ELO) an einem Längsende der Vertiefung (VT) aufweist, bevorzugt in einer Wand (WD) der Inkubationsrinne, so dass über den Kanal (AKN, EL) Flüssigkeit (FL) in die Vertiefung (VT) eingebracht oder abgesaugt werden kann,
wobei in die Inkubationsrinne ein Träger (TR) eingelegt werden kann, welcher bei Einlegen in die Inkubationsrinne die Probe (PR) auf seiner Unterseite (UTR), welche dem Vertiefungsboden (VB) zugewandt ist, aufweist, so dass die Unterseite des Trägers (TR), der Vertiefungsboden (VB) und die Wände (WD) ein Kompartiment (KAV) für die Flüssigkeit (FL) bilden.

12. System nach Anspruch 11,
wobei die Inkubationsrinne Mittel (AF) zum Fixieren eines Abstandes zwischen der Unterseite (UT) des Trägers und dem Vertiefungsboden (VB) aufweist, so dass die Unterseite des Trägers, der Vertiefungsboden und die Wände ein Kompartiment für die Flüssigkeit bilden.

## Claims

1. An incubation device (V),
- comprising a multiplicity of rocking platforms (W), which are respectively mounted tiltably about a respective axis (A), a respective rocking platform (W) comprising a respective reception device (AN) for mechanically reversible reception of a respective incubation tray (I),
- furthermore comprising a common drive unit (AE) for common generation of respective rocking movements of the respective rocking platforms, in particular from a neutral position (NL), through a particular angle range (WB) on the basis of a drive movement of the drive unit (AE),
wherein each of the respective rocking platforms (W) is coupled to the drive unit (AE) by means of a respective coupling mechanism (K),
and wherein each of the respective coupling mechanisms (K) elastically resiliently couples the respective rocking platform (W) to the drive unit (AE) in such a way that, when the rocking platform (W) is restrained, the rocking platform (W) remains in a fixed position despite continuation of the drive movement, but furthermore, after the restraint of the rocking platform (W) is ended, the rocking platform (W) again carries out the rocking movement through the particular angle range (WB) because of the drive movement.

2. The incubation device as claimed in claim 1,
wherein the coupling mechanism (K) comprises at least one elastic spring element (F), which is configured in such a way that the spring element (F) is elastically deformed in the event of application of a holding force to restrain the rocking platform (W) and simultaneous continuation of the drive movement.

3. The incubation device as claimed in claim 2,
wherein the rocking platform (W) comprises a force reception element (KA) arranged eccentrically with respect to the axis (A) of the rocking platform (W), the deflection of which about the axis midpoint (M) causes tilting of the rocking platform (W),
wherein the coupling mechanism (K) furthermore comprises at least one pusher element (MI) which, because of the drive movement of the drive unit (AE), generates a force on the force reception element (KA) to deflect the force reception element (KA),
and wherein a force transmission from the pusher element (MI) to the force reception element (KA) is coupled to the spring element (F).

4. The incubation device as claimed in claim 1,
furthermore comprising a holding device (H) for restraining at least one of the rocking platforms (W) in the fixed position.

5. The incubation device as claimed in claim 4,
wherein the drive unit (AE) is arranged below the rocking platform (W), wherein the holding device (H) comprises on its lower side an engagement element (EE) having an essentially convex geometrical shape (KOF),
wherein the rocking platform (W) furthermore comprises on its upper side an engagement recess (EM) having an essentially concave geometrical shape (KOF), which corresponds with the convex geometrical shape of the engagement element (EE),
and wherein movement of the holding device (H) from above in the direction of the upper side of the rocking platform (W) causes engagement of the engagement element (EE) in the engagement recess (EM) in order to prevent the rocking movement of the rocking platform (W) in the fixed position.

6. The incubation device as claimed in claim 2,
wherein the rocking platform (W) furthermore comprises a metal element (MT), which is firmly mechanically coupled to the rocking platform (W) and carries out the rocking movement together with the rocking platform (W), wherein the incubation device (V) furthermore comprises an electromagnet (ELM) and a control unit (SE),
wherein the control unit (SE) is configured to drive the holding device (H) in such a way that the holding device (H) restrains the rocking platform (W) in the fixed position, in particular by means of engagement of the engagement element (EE) in the engagement recess (EM), and furthermore subsequently to activate the electromagnet (ELM) so that a magnetic force between the electromagnet (ELM) and the metal element (MT) also restrains the rocking platform (W) in the fixed position when the holding device (H) no longer restrains the rocking platform (W).

7. The incubation device as claimed in claim 1,
wherein the reception device (AN) is configured with an upwardly open opening (OF) on an upper side of the rocking platform (W), into which the incubation tray (I) can be inserted.

8. The incubation device as claimed in claim 7,
wherein the reception device (AN) comprises a bottom (B) on which a lower side (U) of the incubation tray (I) bears in the inserted state,
and wherein the reception device (AN) furthermore comprises a clamping mechanism (KL1, KL2) in order to hold the lower side (U) of the incubation tray (I) on the bottom (B).

9. The incubation device as claimed in claim 8,
wherein the rocking platform (W) furthermore comprises a heating device (HZ) for heating the bottom (B).

10. A system,
comprising an incubation device (V) as claimed in one of claims 1 to 9 and at least one incubation tray (I) which can be placed in a rocking platform (W).

11. The system as claimed in claim 10,
wherein the incubation tray (I) comprises an indentation (VT), formed by the walls (WD) of the incubation tray (I), having an indentation bottom (VB),
wherein the incubation tray (I) comprises at least one opening (ALO, ELO) which opens into a channel (AKN, EK) at a longitudinal end of the indentation (VT), preferably in a wall (WD) of the incubation tray, so that liquid (FL) can be introduced into the indentation (VT), or aspirated, through the channel (AKN, EL),
wherein a carrier (TR) may be placed in the incubation tray, which carrier comprises the sample (PR) on its lower side (UTR), which faces toward the indentation bottom (VB), when it is placed in the incubation tray, so that the lower side of the carrier (TR), the indentation bottom (VB) and the walls (WD) form a compartment (KAV) or the liquid (FL).

12. The system as claimed in claim 11,
wherein the incubation tray comprises means (AF) for fixing a distance between the lower side (UT) of the carrier and the indentation bottom (VB) such that the lower side of the carrier, the indentation bottom and the walls form a compartment for the liquid.

## Revendications

1. Dispositif d'incubation (V),
- comportant une pluralité de tables basculantes (W) qui sont chacune montées de manière pivotante sur un axe respectif (A), une table basculante respective (W) comportant un dispositif de réception respectif (AN) destiné à recevoir de manière mécaniquement réversible un conduit d'incubation respectif (I),
- comportant en outre une unité d'entraînement commune (AE) destinée à effectuer conjointement des mouvements de basculement respectifs des tables basculantes respectives, en particulier à partir d'une position zéro (NL), sur une certaine plage angulaire (WB) en raison d'un mouvement d'entraînement de l'unité d'entraînement (AE),
chacune des tables basculantes respectives (W) étant accouplée à l'unité d'entraînement (AE) par le biais d'un mécanisme d'accouplement respectif (K),
et chacun des mécanismes d'accouplement respectifs (K) accouplant la table basculante respective (W) à l'unité d'entraînement (AE) d'une manière élastique de sorte que, lorsque la table basculante (W) est immobilisée, la table basculante (W) reste dans une position fixe malgré la poursuite du mouvement d'entraînement, de plus, cependant, après omission d'immobiliser la table basculante (W), la table basculante (W) exécute à nouveau le mouvement de basculement sur la plage angulaire spécifique (WB) en raison du mouvement d'entraînement.

2. Dispositif d'incubation selon la revendication 1,
le mécanisme d'accouplement (K) comportant au moins un élément à ressort élastique (F) qui est conçu de telle sorte que, lorsqu'une force de retenue est appliquée pour immobiliser la table basculante (W) et que le mouvement d'entraînement se poursuit, l'élément à ressort (F) est déformé élastiquement.

3. Dispositif d'incubation selon la revendication 2,
la table basculante (W) comportant un élément d'absorption de force (KA) qui est disposé excentriquement par rapport à l'axe (A) de la table basculante (W) et dont la déviation autour du point central de l'axe (M) entraîne un pivotement de la table basculante (W),
le mécanisme d'accouplement (K) comportant également au moins un élément entraîneur (MI) qui, en raison du mouvement d'entraînement de l'unité d'entraînement (AE), provoque une force sur l'élément de réception de force (KA) pour dévier l'élément de réception de force (KA)
et une transmission de force de l'élément entraîneur (MI) à l'élément de réception de force (KA) étant accouplée à l'élément à ressort (F).

4. Dispositif d'incubation selon la revendication 1,
comprenant en outre un dispositif de retenue (H) destiné à immobiliser l'une au moins des tables basculantes (W) dans la position fixe.

5. Dispositif d'incubation selon la revendication 4,
l'unité d'entraînement (AE) étant disposée au-dessous de la table basculante (W),
le dispositif de retenue (H) comportant sur son côté inférieur un élément d'engagement (EE) ayant une forme géométrique sensiblement convexe (KOF),
la table basculante (W) comportant également sur son côté supérieur un creux d'engagement (EM) ayant une forme géométrique sensiblement concave (KOF) qui correspond à la forme géométrique convexe de l'élément d'engagement (EE),
et un déplacement du dispositif de retenue (H) depuis le haut en direction du côté supérieur de la table basculante (W) amenant l'élément d'engagement (EE) à s'engager dans le creux d'engagement (EM) pour empêcher le mouvement de basculement de la table basculante (W) dans la position fixe.

6. Dispositif d'incubation selon la revendication 2,
la table basculante (W) comportant également un élément métallique (MT) qui est accouplé mécaniquement de manière fixe à la table basculante (W) et qui effectue le mouvement de basculement conjointement avec la table basculante (W),
le dispositif d'incubation (V) comportant en outre un électro-aimant (ELM) et une unité de commande (SE),
l'unité de commande (SE) étant conçue pour commander le dispositif de retenue (H) de telle sorte que le dispositif de retenue (H) immobilise la table basculante (W) dans la position fixe, notamment au moyen d'un engagement de l'élément d'engagement (EE) dans l'évidement d'engagement (EM) puis pour activer l'électroaimant (ELM) de telle sorte qu'une force magnétique entre l'électroaimant (ELM) et l'élément métallique (MT) immobilise la table basculante (W) dans la position fixe même si le dispositif de retenue (H) n'immobilise plus la table basculante (W).

7. Dispositif d'incubation selon la revendication 1,
le dispositif de réception (AN) étant formé, sur un côté supérieur de la table basculante (W), avec une ouverture (OF) qui est ouverte en haut et dans laquelle le conduit d'incubation (I) peut être inséré.

8. Dispositif d'incubation selon la revendication 7,
le dispositif de réception (AN) comportant un fond (B) sur lequel un côté inférieur (U) du conduit d'incubation (I) vient reposer à l'état inséré,
et le dispositif de réception (AN) comportant en outre un mécanisme de serrage (KL1, KL2) afin de maintenir le côté inférieur (U) du conduit d'incubation (I) sur le fond (B).

9. Dispositif d'incubation selon la revendication 8,
la table basculante (W) comportant en outre un dispositif de chauffage (HZ) destiné à chauffer le fond (B).

10. Système
comportant un dispositif d'incubation (V) selon l'une des revendications 1 à 9 et au moins un conduit d'incubation (I) pouvant être placé dans une table basculante (W).

11. Système selon la revendication 10,
le conduit d'incubation (I) comportant une dépression (VT) formée par les parois (WD) du conduit d'incubation (I) et pourvue d'un fond de dépression (VB),
le conduit d'incubation (I) comportant au moins une ouverture (ALO, ELO), débouchant dans un conduit (AKN, EK), à une extrémité longitudinale de la dépression (VT), de préférence dans une paroi (WD) du conduit d'incubation, de sorte que du liquide (FL) puisse être introduit ou aspiré dans la dépression (VT) par le biais du conduit (AKN, EL),
un support (TR) pouvant être inséré dans le conduit d'incubation et comportant, lorsqu'il est inséré dans le conduit d'incubation, l'échantillon (PR) sur son côté inférieur (UTR) dirigé vers le fond (VB) de la dépression de sorte que le côté inférieur du support (TR), le fond (VB) la dépression et les parois (WD) forment un compartiment (KAV) destiné au liquide (FL).

12. Système selon la revendication 11,
le conduit d'incubation comportant des moyens (AF) pour fixer une distance entre le côté inférieur (UT) du support et le fond (VB) de la dépression de sorte que le côté inférieur du support, le fond de la dépression et les parois forment un compartiment destiné au liquide.
